# EUROPEAN PATENT APPLICATION

(11) **EP 1 405 645 A1**
(43) Date of publication of application: **07.04.2004**
(21) Application number: 02741233.7
(22) Date of filing: 21.06.2002
(51) Int. Cl.: A61K 45/00, A61K 38/17, A61P 37/00, A61P 11/00, A61P 17/00, A61P 1/00, A61P 35/00, A61P 33/00

(54) **REMEDIES FOR EOSINOPHILIC DISEASES**

(30) Priority: 22.06.2001 JP 2001190083
(71) Applicant: Daiichi Suntory Biomedical Research Limited, Mishima-gun, Osaka 618-8503 (JP); Daiichi Suntory Pharma Co., Ltd., Tokyo 102-0083 (JP)
(72) Inventor: MIURA, Kenju, Toyonaka-shi, Osaka 560-0011 (JP); MATSUBAYASHI, Maki, Kawasaki-shi, Kanagawa 214-0011 (JP); SAITO, Hirohisa, Wako-shi, Saitama 351-0114 (JP); MATSUMOTO, Kenji, Setagaya-ku, Tokyo 154-0001 (JP)
(74) Representative: HOFFMANN - EITLE
(86) International application number: PCT/JP2002/006219
(87) International publication number: WO 2003/000286

(57) **Abstract**

Herein disclosed are therapeutic agents for hypereosinophilic diseases comprising a substance inducing eosinophil apoptosis via CD30 and/or a molecule involved in CD30 signal transduction as an active ingredient. Such therapeutic agents are effective for allergic diseases, respiratory diseases, skin diseases, autoimmune diseases, immunodeficiency diseases, digestive diseases, neoplastic diseases and parasitic infections.

## Description

### TECHNICAL FIELD

The present invention relates to therapeutic agents or methods or other related means for hypereosinophilic diseases caused by increased eosinophils in peripheral blood or tissue, comprising a substance inducing eosinophil apoptosis via CD30 and/or a molecule involved in CD30 signal transduction as an active ingredient.

### BACKGROUND ART

Inflammation is a pathologic state in which a plurality of inflammatory cells such as mast cells, lymphocytes, eosinophils and basophils form a complex network via a plurality of mediators to cause tissue damages. Conventional therapies for inflammation, especially allergic inflammation were focused on the inhibition of tissue reactions caused by specific IgE-mediated degranulation of mast cells or basophils/ release of chemical mediators, i.e. chemotaxis, infiltration and activation of inflammatory cells. Hyposensitization therapy, degranulation inhibitors/ chemical mediator release inhibitors, and chemical mediator antagonists have greatly contributed to the treatment of allergic inflammation. Allergy therapies are centered on the inhibition of this IgE-dependent mechanism, and new chemical mediators, especially eicosanoid synthesis inhibitors/antagonists are still now being extensively developed.

On the other hand, recent studies showed that eosinophil granule proteins directly induce tissue damages or inflammation, indicating that eosinophils play a key role in inflammatory response, especially allergic inflammation. In the pathology of inflammation eosinophils are known to show "increased production in bone marrow", "selective infiltration into inflammatory region and activation", and "prolonged survival". As a result, surviving activated eosinophils increase to invite serious tissue damages by continuous release of cytotoxic eosinophil granule proteins. Clinical findings also show increased peripheral blood eosinophils in e.g. patients with chronic bronchial asthma. It has also been reported that the number of peripheral blood eosinophils or the number of eosinophils in bronchoalveolar lavage fluid correlates with bronchial hypersensitivity and that the number of peripheral blood eosinophils is normalized as the condition improves. Thus, the regulation of eosinophils is also one of important goals of inflammation therapies because a major symptom of various diseases such as allergic diseases, parasitic infections and autoimmune diseases is the inflammatory response.

Studies on inflammation therapies targeting eosinophils have been so far directed to the "inhibition of increased production in bone marrow", "inhibition of selective infiltration into tissue", "inhibition of chemotaxis", "inhibition of activation (degranulation)", "inactivation of granule proteins (MBP, EPO, ECP, EDN) or active oxygen species", etc., and clinical application of blocking of the signal pathway mediated by an eosinophilopoietic cytokine IL-5 (anti-IL-5R antibodies, soluble IL-5R or anti-IL-5 antibodies) to allergic inflammation is currently under development. However, any therapy targeting eosinophils has not been clinically established to date.

Apoptosis induction in eosinophils is very effective in terminating inflammatory signaling because the survival of eosinophils is extended by the inhibition of apoptosis and increased activated eosinophils undergo necrosis to prolong inflammation by granule protein secretion at inflamed sites and eosinophils expressing little Bcl-2 responsible for apoptosis inhibition are readily led to apoptosis by removing survival-promoting cytokines (IL-3, IL-5, GM-CSF, IFN-γ, etc.). In other words, it is important to induce apoptosis in eosinophils to terminate inflammation.

Therapeutic applications of factors capable of inducing apoptosis in eosinophils such as TGF-β, anti-Fas antibodies and FasL have been proposed. It has been suggested that apoptosis induction in eosinophils is a part of anti-inflammatory effects of steroids (J. Clin. Invest. 88: 1982-1987, 1991). However, some reports showed that administration of anti-Fas antibodies has the problem of side effects because they induce fulminant hepatitis (Cell, 88: 355-365, 1997) or that apoptosis induction in eosinophils by steroids is diminished by IL-5 or the like (J. Clin. Invest. 88: 1982-1987, 1991). Thus, there has not existed so far any therapeutic agent or method effective for hypereosinophilic diseases by apoptosis induction in eosinophils.

On the other hand, CD30 was identified as a cell surface molecule recognized by a monoclonal antibody Ki-1 prepared against Reed-Sternberg cell line L428 derived from Hodgkin's disease as an antigen in 1982 and reported as an antigen specific for Hodgkin's disease cells (Nature, 299: 65, 1982). Subsequently, this molecule was shown to be expressed in some non-Hodgkin's lymphoma, anaplastic large cell lymphoma, tumor cells such as malignant melanoma and mesenchymal chondrosarcoma cells, various cell lines, mitogen-activated T and B cells, T and B cells transformed by viruses (HIV, HTLV-I, -II, EBV), activated macrophages, activated NK cells, uterine decidua, etc. Previous analyses reported that signals from CD30 show a wide range of effects such as the growth and cell death of T cells and increased production of cytokines (Blood, 83: 2045, 1994), but the mechanism remain mostly unknown. In CD30-deficient mice, a dramatic increase was observed in the number of CD4+8+ double positive thymocytes, suggesting the inhibition of negative selection for thymocytes (self-reactive T cells) (J. Exp. Med., 187: 427-432, 1998). Another report showed that certain monoclonal antibodies recognizing a CD30 ligand-binding site themselves have an antitumor effect against anaplastic large cell lymphoma. This suggests that CD30-mediated signaling induces apoptosis in certain cells (Clinical Immunology, 34: 67-71, 2000). On the basis of the structure of its extracellular domains, the CD30 molecule was shown to be a member of the TNF receptor (TNFR) superfamily and a CD30 ligand (CD153) was shown to be a member of the TNF superfamily. It was also reported that cytoplasmic domains of CD30 have no death domains as found in Fas but include two TNFR-associated factor (TRAF)-binding sites to activate NF-kB via the TRAF molecule (Int. Immunol. 10: 203, 1998).

A recent report further showed that CD30 signaling depend on not only the binding of their ligands but also their expression levels, i.e. they are self-activated by overexpression per se to ligand-independently activate NF-kB (Clinical immunology, 34: 812-819, 2000). The expression level of CD30 considerably varies among cell types and activation and differentiation stages, indicating that various effects of CD30 seem to partly result from variations in the expression level, but they cannot be explained only by the expression level in many cases, suggesting that their effects on cells may also be modified by variations in the intracellular impulse conducting system. Elevated serum levels of soluble CD30 are associated with the prognosis in patients with Hodgkin's disease, AIDS, type-B hepatitis, atopic disease or Omenn syndrome or patients with autoimmune diseases such as rheumatoid arthritis and systemic lupus erythematosus (Proc. Natl. Acad. Sci. U.S.A., 94: 8670-8674, 1997, J. Neuroimmunol., 97, 182-190, 1999, J. Neurol. Sci., 171: 49-55, 1999, Clin. Endocrinol., 49: 609-613, 1998, Br. J. Dermatol., 140: 73-78, 1999). Especially, raised serum soluble CD30 levels in patients with Hodgkin's disease and a correlation between serum ECP (Eosinophil Cationic Protein) levels in atopic dermatitis or stages or prognosis of AIDS and serum soluble CD30 levels suggest involvement of CD30 in these diseases.

In Hodgkin's disease, an increase in eosinophils was also reported (Ann. Oncol., 8: 89-96, 1997, Blood, 95: 1207-1213, 2000), but the cause and mechanism are unknown and no report shows that CD30 is expressed in eosinophils. Thus, no report has so far associated the increase in eosinophils with raised soluble CD30 levels.

To terminate inflammation, it is necessary to inhibit new production of eosinophils or their infiltration into inflammatory region and activation and to eliminate activated eosinophils. If eosinophils are lyzed in the course of necrosis, however, tissues are further damaged by granule protein secretion due to the destruction of cell membranes and inflammation becomes aggravated. Therefore, the present invention aims to provide therapeutic agents or methods or related means for hypereosinophilic diseases using a substance inducing rapid and strong apoptosis in eosinophils.

### DISCLOSURE OF THE INVENTION

The present invention proposes to overcome the above-described problems of the prior art. We accomplished the present invention on the basis of the finding that CD30-mediated signaling via anti-human CD30 monoclonal antibodies or the like can induce rapid and strong apoptosis in eosinophils to effectively treat hypereosinophilic diseases including allergic diseases.

Prior to the disclosure by the present invention it was not known that the CD30 molecule is expressed in human peripheral blood eosinophils and that CD30-mediated signaling has a physiological activity inducing apoptosis in eosinophils.

We pursued our studies on the hypothesis that CD30 regulates apoptosis of eosinophils and that if apoptosis induction is inhibited by soluble CD30, diseases caused by increased eosinophils or eosinophilia will occur. As a result, we accomplished the present invention on the basis of the finding that CD30-mediated signaling can induce apoptosis selectively in human peripheral blood eosinophils more rapidly and strongly than known signaling and that this apoptosis induction does not simply result from the antagonism of IL-5-mediated survival signaling but occurs via a pathway independent of IL-5 signal transduction. As shown in detail in the examples below, we found that apoptosis was induced as rapidly as within 1-4 hours in 50% or more of human peripheral blood eosinophils cultured on plates to which had been immobilized certain anti-human CD30 monoclonal antibodies recognizing a CD30 ligand-binding site.

Thus, we accomplished the present invention on the basis of the finding that a substance inducing eosinophil apoptosis via CD30 and/or a molecule involved in CD30 signal transduction could be a therapeutic agent for hypereosinophilic diseases.

Accordingly, the present invention relates to:
(1) a therapeutic agent for a hypereosinophilic disease comprising a substance inducing eosinophil apoptosis via CD30 and/or a molecule involved in CD30 signal transduction as an active ingredient;
(2) the therapeutic agent for a hypereosinophilic disease as defined in (1) above wherein the substance inducing eosinophil apoptosis via CD30 and/or a molecule involved in CD30 signal transduction is an immunoglobulin recognizing site recognized by Ber-H8 or HRS-4 or an active fragment, peptide or low-molecular weight compound thereof;
(3) the therapeutic agent for a hypereosinophilic disease as defined in (1) wherein the substance inducing eosinophil apoptosis via CD30 and/or a molecule involved in CD30 signal transduction is an immunoglobulin or an active fragment thereof (including those derived from natural sources or obtained by gene recombination);
(4) the therapeutic agent for a hypereosinophilic disease as defined in (2) above wherein the substance inducing eosinophil apoptosis via CD30 and/or a molecule involved in CD30 signal transduction is an immunoglobulin recognizing at least a part or the whole of the amino acid sequence of SEQ ID NO: 1 (the amino acid sequence between positions 112 and 412 of the amino acid sequence of CD30) or an active fragment thereof;
(5) the therapeutic agent for a hypereosinophilic disease as defined in (4) above wherein the immunoglobulin is Ber-H8 or HRS-4;
(6) the therapeutic agent for a hypereosinophilic disease as defined in (1) above wherein the substance inducing eosinophil apoptosis via CD30 and/or a molecule involved in CD30 signal transduction is a CD30 ligand or an active fragment thereof (including those derived from natural sources or obtained by gene recombination);
(7) the therapeutic agent for a hypereosinophilic disease as defined in (6) above wherein the CD30 ligand is CD153;
(8) the therapeutic agent for a hypereosinophilic disease as defined in (1) above wherein the substance inducing eosinophil apoptosis via CD30 and/or a molecule involved in CD30 signal transduction is a nucleic acid encoding a CD30 ligand or an active fragment thereof;
(9) the therapeutic agent for a hypereosinophilic disease as defined in (8) above wherein the nucleic acid is a DNA encoding the amino acid of SEQ ID NO: 2 (the amino acid sequence of CD153);
(10) the therapeutic agent for a hypereosinophilic disease as defined in (1) above wherein the substance inducing eosinophil apoptosis via CD30 and/or a molecule involved in CD30 signal transduction is a phagocytic cell expressing and/or secreting a CD30 ligand;
(11) the therapeutic agent for a hypereosinophilic disease as defined in (1) above wherein the substance inducing eosinophil apoptosis via CD30 and/or a molecule involved in CD30 signal transduction is a substance allowing a phagocytic cell to express and/or secret a CD30 ligand;
(12) the therapeutic agent for a hypereosinophilic disease as defined in (1) above wherein the substance inducing eosinophil apoptosis via CD30 and/or a molecule involved in CD30 signal transduction is a substance capable of regulating CD30 expression and/or aggregation;
(13) the therapeutic agent for a hypereosinophilic disease as defined in any one of (1) to (12) above wherein the hypereosinophilic disease is a disease selected from the group consisting of allergic disease, respiratory disease, skin disease, autoimmune disease, immunodeficiency disease, digestive disease, neoplastic disease, parasitic infection or idiopathic hypereosinophilic syndrome;
(14) the therapeutic agent for a hypereosinophilic disease as defined in (13) above wherein the allergic disease is a disease selected from the group consisting of drug allergy, bronchial asthma and allergic rhinitis;
(15) the therapeutic agent for a hypereosinophilic disease as defined in (13) above wherein the respiratory disease is eosinophilic pulmonary infiltration;
(16) the therapeutic agent for a hypereosinophilic disease as defined in (13) above wherein the skin disease is a disease selected from the group consisting of atopic dermatitis, urticaria, angioedema, psoriasis, Kimura's disease, Episodic angioedema with eosinophilia, eosinophilic pustular folliculitis and lymphomatoid papulosis;
(17) the therapeutic agent for a hypereosinophilic disease as defined in (13) above wherein the autoimmune disease is a disease selected from the group consisting of polyarteritis, rheumatoid arthritis and systemic lupus erythematosus;
(18) the therapeutic agent for a hypereosinophilic disease as defined in (13) above wherein the immunodeficiency disease is a disease selected from the group consisting of hyper IgE syndrome, Wiscott-Aldrich syndrome and Omenn syndrome;
(19) the therapeutic agent for a hypereosinophilic disease as defined in (13) above wherein the digestive disease is a disease selected from the group consisting of allergic gastroenteritis, eosinophilic gastroenteritis, ulcerative colitis and pancreatitis;
(20) the therapeutic agent for a hypereosinophilic disease as defined in (13) above wherein the tumorous disease is Hodgkin's disease;
(21) the therapeutic agent for a hypereosinophilic disease as defined in (13) above wherein the parasitic infection is a disease selected from the group consisting of anisakiasis, trichiniasis, strongyloidiasis, schistosomiasis japonica and pulmonary distomiasis;
(22) a therapeutic method for a hypereosinophilic disease, comprising administering a substance inducing eosinophil apoptosis via CD30 and/or a molecule involved in CD30 signal transduction;
(23) the therapeutic method for a hypereosinophilic disease as defined in (22) above wherein the substance inducing eosinophil apoptosis via CD30 and/or a molecule involved in CD30 signal transduction is an immunoglobulin recognizing a site recognized by Ber-H8 or HRS-4 or an active fragment or low-molecular weight compound thereof;
(24) the therapeutic method for a hypereosinophilic disease as defined in (22) above wherein the substance inducing eosinophil apoptosis via CD30 and/or a molecule involved in CD30 signal transduction is an immunoglobulin or an active fragment thereof (including those derived from natural sources or obtained by gene recombination);
(25) the therapeutic method for a hypereosinophilic disease as defined in (23) above wherein the substance inducing eosinophil apoptosis via CD30 and/or a molecule involved in CD30 signal transduction is an immunoglobulin recognizing at least a part or the whole of the amino acid sequence of SEQ ID NO: 1 (the amino acid sequence between positions 112 and 412 of the amino acid sequence of CD30) or an active fragment thereof;
(26) the therapeutic method for a hypereosinophilic disease as defined in (24) above wherein the immunoglobulin is Ber-H8 or HRS-4;
(27) the therapeutic method for a hypereosinophilic disease as defined in (22) above wherein the substance inducing eosinophil apoptosis via CD30 and/or a molecule involved in CD30 signal transduction is a CD30 ligand or an active fragment thereof (including those derived from natural sources or obtained by gene recombination);
(28) the therapeutic method for a hypereosinophilic disease as defined in (27) above wherein the CD30 ligand is CD153;
(29) the therapeutic method for a hypereosinophilic disease as defined in (22) above wherein the substance inducing eosinophil apoptosis via CD30 and/or a molecule involved in CD30 signal transduction is a nucleic acid encoding a CD30 ligand or an active fragment thereof;
(30) the therapeutic method for a hypereosinophilic disease as defined in (29) above wherein the nucleic acid is a DNA encoding the amino acid of SEQ ID NO: 2 (the amino acid sequence of CD153);
(31) the therapeutic method for a hypereosinophilic disease as defined in (22) above wherein the substance inducing eosinophil apoptosis via CD30 and/or a molecule involved in CD30 signal transduction is a phagocytic cell expressing and/or secreting a CD30 ligand;
(32) the therapeutic method for a hypereosinophilic disease as defined in (22) above wherein the substance inducing eosinophil apoptosis via CD30 and/or a molecule involved in CD30 signal transduction is a substance allowing a phagocytic cell to express and/or secret a CD30 ligand;
(33) the therapeutic method for a hypereosinophilic disease as defined in (22) above wherein the substance inducing eosinophil apoptosis via CD30 and/or a molecule involved in CD30 signal transduction is a substance capable of regulating CD30 expression and/or aggregation;
(34) the therapeutic method for a hypereosinophilic disease as defined in any one of (22) to (33) above wherein the hypereosinophilic diseases is a disease selected from the group consisting of allergic disease, respiratory disease, skin disease, autoimmune disease, immunodeficiency disease, digestive disease, neoplastic disease, parasitic infection or idiopathic hypereosinophilic syndrome;
(35) the therapeutic method for a hypereosinophilic disease as defined in (34) above wherein the allergic disease is a disease selected from the group consisting of drug allergy, bronchial asthma and allergic rhinitis;
(36) the therapeutic method for a hypereosinophilic disease as defined in (34) above wherein the respiratory disease is eosinophilic pulmonary infiltration;
(37) the therapeutic method for a hypereosinophilic disease as defined in (34) above wherein the skin disease is a disease selected from the group consisting of atopic dermatitis, urticaria, angioedema, psoriasis, Kimura's disease, Episodic angioedema with eosinophilia, eosinophilic pustular folliculitis and lymphomatoid papulosis;
(38) the therapeutic method for a hypereosinophilic disease as defined in (34) above wherein the autoimmune disease is a disease selected from the group consisting of polyarteritis, rheumatoid arthritis and systemic lupus erythematosus;
(39) the therapeutic method for a hypereosinophilic disease as defined in (34) above wherein the immunodeficiency disease is a disease selected from the group consisting of hyper IgE syndrome, Wiscott-Aldrich syndrome and Omenn syndrome;
(40) the therapeutic method for a hypereosinophilic disease as defined in (34) above wherein the digestive disease is a disease selected from the group consisting of allergic gastroenteritis, eosinophilic gastroenteritis, ulcerative colitis and pancreatitis;
(41) the therapeutic method for a hypereosinophilic disease as defined in (34) above wherein the tumorous disease is Hodgkin's disease;
(42) the therapeutic method for a hypereosinophilic disease as defined in (34) above wherein the parasitic infection is a disease selected from the group consisting of anisakiasis, trichiniasis, strongyloidiasis, schistosomiasis japonica and pulmonary distomiasis;
(43) a diagnostic method for a hypereosinophilic disease comprising measuring the serum soluble CD30 level to diagnose the disease on the basis of variations in the level; and
(44) a screening method for a CD30-mediated eosinophil apoptosis inducer comprising the steps of: (a) primary screening based on the inhibition of binding of anti-CD30 antibodies to CD30-expressing cells, (b) secondary screening based on apoptosis induction in peripheral blood-, cord blood- or marrow-derived cultured eosinophils, and (c) tertiary screening based on apoptosis induction in peripheral blood eosinophils.

### BRIEF EXPLANATION OF THE DRAWINGS

FIG. 1 is a graph showing the results of an analysis of CD30 expression in human peripheral blood eosinophils using 6 mouse anti-human CD30 monoclonal antibodies or a control mouse IgG.
FIG. 2 is a graph showing the results of an analysis of CD30 expression in human peripheral blood eosinophils activated by IL-5 using 6 mouse anti-human CD30 monoclonal antibodies or a control mouse IgG.
FIG. 3 is an electrophoretogram showing the results of an analysis of CD30 mRNA expression in human peripheral blood eosinophils.
FIG. 4 is a graph showing the results of an apoptosis induction assay in human peripheral blood eosinophils by anti-human CD30 monoclonal antibodies.
FIG. 5 is a graph showing the results of an evaluation of individual variations among eosinophil donors in the apoptosis induction in human peripheral blood eosinophils by anti-human CD30 monoclonal antibodies.
FIG. 6 is a graph showing changes with time in the apoptosis induction in human peripheral blood eosinophils by anti-human CD30 monoclonal antibodies HRS-4 and Ber-H8.
FIG. 7 is an electrophoretogram showing the results of an evaluation of apoptosis induction in human peripheral blood eosinophils by anti-human CD30 monoclonal antibodies HRS-4 and Ber-H8 based on DNA fragmentation.
FIG. 8 is a graph showing the results of an evaluation of the ability of anti-human CD30 monoclonal antibodies HRS-4 and Ber-H8 to induce apoptosis in human peripheral blood eosinophils in comparison with an anti-human Fas monoclonal antibody.
FIG. 9 is a graph showing the results of an evaluation of the ability of anti-human CD30 monoclonal antibodies HRS-4 and Ber-H8 to induce apoptosis in neutrophils.
FIG. 10 is a graph showing the results of an evaluation of the ability of anti-human CD30 monoclonal antibodies HRS-4 and Ber-H8 to induce apoptosis in lymphocytes.
FIG. 11 is a graph showing the results of an evaluation of the ability of anti-human CD30 monoclonal antibodies HRS-4 and Ber-H8 to induce apoptosis in cultured human mast cells.
FIG. 12 is a graph showing the influence of high-concentration IL-5 on the apoptosis induction in human peripheral blood eosinophils by anti-human CD30 monoclonal antibodies HRS-4 and Ber-H8.
FIG. 13 is a graph showing the results of an evaluation of apoptosis induction in human peripheral blood eosinophils by an anti-mouse CD30 monoclonal antibody (YMSM636.4.10).
FIG. 14 is a graph showing the results of an evaluation of apoptosis induction in human peripheral blood eosinophils by a mouse CD30 ligand.
FIG. 15 is a graph showing the results of an evaluation of apoptosis induction in human peripheral blood eosinophils by anti-human CD30 monoclonal antibodies HRS-4 and Ber-H8 immobilized on polystyrene microbeads.
FIG. 16 is a graph showing the results of a phagocytosis assay using cord blood-derived macrophages on human peripheral blood eosinophils in which apoptosis was induced by anti-human CD30 monoclonal antibodies HRS-4 and Ber-H8 immobilized on polystyrene microbeads.
FIG. 17 is a graph showing the results of a phagocytosis assay using PMA-stimulated cells of a human mononuclear cell line U937 on human peripheral blood eosinophils in which apoptosis was induced by anti-human CD30 monoclonal antibodies HRS-4 and Ber-H8 immobilized on polystyrene microbeads.

### THE MOST PREFERRED EMBODIMENTS OF THE INVENTION

As used herein, the molecule involved in CD30 signal transduction means not only a molecule directly or indirectly acting on CD30 expressed on the surfaces of eosinophils but also a molecule involved in CD30-mediated intracellular signal transduction as well as a molecule involved in each step of cellular signal transduction at the level of cell surfaces to nuclei. Therefore, the substance inducing eosinophil apoptosis via CD30 and/or a molecule involved in CD30 signal transduction as used herein means a substance directly or indirectly acting on each molecule in such various steps of extracellular or intracellular signaling to induce eosinophil apoptosis.

The substance inducing eosinophil apoptosis via CD30 and/or a molecule involved in CD30 signal transduction is not only an anti-CD30 antibody but may also be any substance that can induce apoptosis in eosinophils and satisfy necessary conditions for clinical applications. Examples are CD30 ligands, phagocytic cells expressing and/or secreting CD30 ligands, substances capable of regulating CD30 expression and/or aggregation, substances allowing phagocytic cells to express and/or secrete CD30 ligands or substances inducing CD30-mediated apoptosis in eosinophils obtained by the screening method described below.

Phagocytic cells expressing and/or secreting CD30 ligands, substances capable of regulating CD30 expression and/or aggregation and substances allowing phagocytic cells to express and/or secrete CD30 ligands can also be expected to induce eosinophil apoptosis via CD30 and/or a molecule involved in CD30 signal transduction. The phagocytic cells expressing and/or secreting CD30 ligands, substances capable of regulating CD30 expression and/or aggregation and substances allowing phagocytic cells to express and/or secrete CD30 ligands can be screened by e.g. immunostaining phagocytes with anti-CD30 ligand antibodies or assaying culture supernatants of phagocytes for CD30 ligands by ELISA.

A preferred substance inducing eosinophil apoptosis via CD30 and/or a molecule involved in CD30 signal transduction is an immunoglobulin recognizing a site recognized by an anti-CD30 antibody Ber-H8 or HRS-4 or an active fragment, peptide or low-molecular weight compound thereof. Ber-H8 and HRS-4 are described as mouse anti-human CD30 monoclonal antibodies (Hybridoma 19, 43-48, 2000) and commercially available (Ber-H8 is available from PharMingen, San Diego, CA and HRS-4 is available from Immunotech, Marseilles, France).

An especially preferred substance inducing eosinophil apoptosis via CD30 and/or a molecule involved in CD30 signal transduction is an immunoglobulin recognizing at least a part or the whole of the amino acid sequence of SEQ ID NO: 1 (the amino acid sequence between positions 112 and 412 of the amino acid sequence of CD30) or an active fragment thereof. An especially preferred immunoglobulin is an anti-CD30 monoclonal antibody.

Any sources and types of anti-CD30 monoclonal antibodies can be used so far as they are highly purified, but especially preferred are monoclonal antibodies from mammals.

The animal species of cells producing the monoclonal antibodies may be any mammals including humans or non-human mammals. Monoclonal antibodies from non-human mammals are preferably derived from rabbits or rodents because of the convenience of preparation. Examples of rodents preferably include (but are not limited to) mouse, rat and hamster. Human antibodies prepared from transgenic animals are also preferred examples.

An example of such an anti-CD30 monoclonal antibody is, but not limited to, Ber-H8 or HRS-4. Anti-CD30 monoclonal antibodies can be basically provided by known techniques as follows. A suitable host is immunized with CD30 as an immunizing antigen according to a standard immunization technique, and the resulting immunized cells are fused to known parent cells by a standard cell fusion technique, and then the fused cells are screened for monoclonal antibody-producing cells by a standard screening method.

The monoclonal antibodies used in the present invention are not limited to those produced by hybridomas, but may be those artificially modified to reduce the antigenicity of materials heterologous with respect to humans or for other purposes. For example, chimeric antibodies consisting of a variable region of a monoclonal antibody from a non-human mammal such as a mouse, a constant region of a human antibody and <TXF FR=0002 HE=250 WI=080 LX=1100 LY=0300> can be used and such chimeric antibodies can be prepared by known processes for preparing chimeric antibodies, especially gene recombination. Reshaped humanized antibodies can also be used in the present invention. These are obtained by replacing the complementarity-determining regions of a human antibody by the complementarity-determining regions of an antibody from a non-human mammal such as a mouse and typical gene recombination techniques for preparing them are also known. Reshaped humanized antibodies useful for the present invention can be obtained by using these known techniques.

In the present invention, the substance inducing eosinophil apoptosis via CD30 and/or a molecule involved in CD30 signal transduction may be a CD30 ligand or an active fragment thereof. Preferred CD30 ligands include (but are not limited to) human-derived CD153 having the amino acid sequence of SEQ ID NO: 2 and mouse-derived CD153 having the amino acid sequence of SEQ ID NO: 5. The substance inducing eosinophil apoptosis via CD30 and/or a molecule involved in CD30 signal transduction may also be a nucleic acid encoding a CD30 ligand or an active fragment thereof. Preferred nucleic acids encoding a CD30 ligand include (but are not limited to) a DNA encoding the amino acid of SEQ ID NO: 2 (the amino acid sequence of human-derived CD153) and a DNA encoding the amino acid of SEQ ID NO: 5 (the amino acid sequence of mouse-derived CD153).

An example of a screening method for a substance inducing eosinophil apoptosis via CD30 and/or a molecule involved in CD30 signal transduction comprises (but is not limited to) primary screening based on the inhibition of binding of anti-CD30 antibodies (e.g. HRS-4, Ber-H8, YMSM636.4.10, etc.) to CD30-expressing cells (e.g. Jurkat, K562, RD, etc.), secondary screening based on apoptosis induction in peripheral blood-, cord blood- or marrow-derived cultured eosinophils, and tertiary screening based on apoptosis induction in peripheral blood eosinophils.

The presence of an apoptosis-inducing effect can be determined on the basis of whether or not eosinophils show characteristic features of apoptosis, specifically observation of morphologic features such as decreased cell volume, fragmentation of DNA and formation of apoptotic body by transmission electron microscopy, or the viability of eosinophils. The cell volume can be measured by an electronic sizing technique using e.g. a Coulter counter. Fragmentation of DNA between nucleosomes can be detected as DNA ladders using a commercially available ladder detection kit for detecting apoptosis (from e.g. Wako Pure Chemical Industries, Ltd., Osaka, Japan). The cell viability can be evaluated by e.g. mitochondrial dehydrogenase activity using a colorimetric MTT assay. MTT can be replaced by a formazan reagent such as WAT-1 or WST-8. The cell viability can also be determined based on the exclusion of viable cells from staining with trypan blue. Alternatively, apoptotic cells having bound FITC-labeled Annexin V can be measured by a flow cytometer (e.g. FACScan Becton Dickinson) using a commercially available kit such as MEBCYTO-Apoptosis Kit (Medical Biological Laboratories, Nagoya, Japan).

In contrast to cell necrosis involving granule protein secretion, apoptotic eosinophils are phagocytized by phagocytes such as macrophages and removed without injuring surrounding tissues/cells before granule proteins are secreted by the destruction of cell membranes.

Generally, the exclusion of apoptotic cells from the phagocytosis by phagocytes is thought to occur via the following four stages.
1. Migration of phagocytes to the periphery of apoptotic cells: At inflammatory region, phagocytes are already adjacent to apoptotic cells.
2. Recognition of apoptotic cells by phagocytes: Phagocytes recognize phagocytosis marker molecules on apoptotic cell surfaces via phagocytosis receptors. One of the best known phagocytosis marker molecules is phosphatidylserine (PS). As phagocytosis receptors, class B scavenger receptor type I (SR-BI) and LOX-1 (lectin-like oxidized low-density lipoprotein receptor-1) recognizing PS have been identified.
3. Engulfment (phagocytosis) of apoptotic cells by phagocytes: In response to signals from phagocytosis receptors, phagocytosis by phagocytes occurs via an unknown signal transduction mechanism.
4. Transport to intracellular organelles such as lysosome and processing of phagocytized apoptotic cells.

Specific examples of hypereosinophilic diseases include allergic diseases, respiratory diseases, skin diseases, autoimmune diseases, immunodeficiency diseases, digestive diseases, idiopathic hypereosinophilic syndromes, neoplastic diseases (e.g. Hodgkin's disease) and parasitic infections.

Therapeutic agents for hypereosinophilic diseases according to the present invention comprising a substance inducing eosinophil apoptosis via CD30 and/or a molecule involved in CD30 signal transduction screened as above as an active ingredient are prepared with carriers, excipients and other additives used for ordinary formulation.

Carriers or excipients for formulation include e.g. lactose, magnesium stearate, starch, talc, gelatin, agar, pectin, acacia, olive oil, sesame oil, cocoa butter, ethylene glycol and other common additives.

Suitable solid compositions for oral administration include tablets, pills, capsules, powders and granules. In such solid compositions, at least one active ingredient is mixed with at least one inert diluent such as lactose, mannitol, glucose, hydroxypropylcellulose, microcrystalline cellulose, starch, polyvinyl pyrrolidone or magnesium aluminometasilicate. The compositions may conventionally contain additives other than inert diluents, e.g. lubricants such as magnesium stearate; disintegrants such as calcium cellulose glycolate; and solubilizers such as glutamic acid or aspartic acid. Tablets or pills may be coated with a sugar coating such as sucrose, gelatin or hydroxypropyl methylcellulose phthalate, or a film of a gastric soluble or enteric material, or two or more layers. Capsules of absorbable materials such as gelatin are also included.

Liquid compositions for oral administration include pharmaceutically acceptable emulsions, solutions, suspensions, syrups and elixirs, and may contain ordinary inert diluents such as purified water and ethanol. In addition to inert diluents, these compositions may contain adjuvants such as wetting agents or suspending agents, sweetening agents, flavoring agents, aromatics and preservatives.

Injections for parenteral administration include sterile aqueous or nonaqueous solutions, suspensions and emulsions. Aqueous solutions and suspensions contain e.g. water for injection and physiological saline for injection. Nonaqueous solutions and suspensions contain e.g. propylene glycol, polyethylene glycol, vegetable oils such as olive oil, alcohols such as ethanol, and Polysorbate® 80. These compositions may further contain adjuvants such as preservatives, wetting agents, emulsifying agents, dispersing agents, stabilizers (e.g. lactose), and solubilizers (e.g. glutamic acid and aspartic acid). These can be sterilized by ordinary sterilizing methods, such as mechanical sterilization with a microfiltration membrane, heat sterilization such as autoclaving or inclusion of a bactericide. Injections may be solution formulations or freeze-dried formulations to be reconstituted before use. Suitable excipients for freeze-drying include e.g. sugar alcohols or sugars such as mannitol or glucose.

When therapeutic agents of the present invention are used for gene therapy, a nucleic acid of the substance inducing eosinophil apoptosis via CD30 and/or a molecule involved in CD30 signal transduction can be integrated into a virus vector, preferably a lentivirus vector, an adeno-associated virus vector, more preferably an adenovirus vector, or into a known vehicle suitable for gene therapy such as a chemically synthesized liposome, a virus envelope or a complex of a virus envelope and a chemical liposome downstream of a promoter sequence that is functional in host cells such as Cytomegalovirus promoter (CMV promoter).

Therapeutic agents for hypereosinophilic diseases according to the present invention are preferably administered via pharmaceutically common routes such as oral or parenteral routes. When the active ingredient is an antibody, they are normally administered via parenteral routes such as injection (subcutaneous, intravenous, intramuscular or intraperitoneal injection) or percutaneous, mucosal, nasal or pulmonary administration, but may also be orally administered.

The amount of the substance inducing eosinophil apoptosis via CD30 and/or a molecule involved in CD30 signal transduction contained as an active ingredient in formulations of the present invention can be determined depending on the type of the disease to be treated, the severity of the disease, the age of the patient and other factors, but generally can be administered in the range of 0.01 to 500 mg/ml, preferably 0.1 to 200 mg/ml expressed as a final concentration.

### INDUSTRIAL APPLICABILITY

The substances inducing eosinophil apoptosis via CD30 and/or a molecule involved in CD30 signal transduction according to the present invention are useful as therapeutic agents for hypereosinophilic diseases, and CD30-mediated apoptosis induction in eosinophils is useful as a therapy for hypereosinophilic diseases based on a novel mechanism. Examples of such diseases are those associated with inflammatory response as a major symptom in which eosinophils play a key role such as allergic diseases, parasitic infections and autoimmune diseases.

The therapeutic agents for hypereosinophilic diseases according to the present invention can induce apoptosis in human peripheral blood eosinophils much more rapidly and strongly than anti-Fas antibodies previously known to induce eosinophil apoptosis. The apoptosis induction is highly specific for eosinophils and no apoptosis is induced in neutrophils, lymphocytes and mast cells. In addition, the apoptosis induction in eosinophils by the therapeutic agents for hypereosinophilic diseases according to the present invention cannot be diminished by IL-5 or the like. Therefore, the therapeutic agents for hypereosinophilic diseases according to the present invention can be expected as clinically very useful therapeutic agents.

### EXAMPLES

The following examples further illustrate the present invention without, however, limiting the scope of the invention thereto.

### Example 1: Analysis of CD30 expression in human peripheral blood eosinophils

Heparinized human peripheral blood was diluted with PBS and layered over Percoll (density 1.090 g/ml, Pharmacia, Uppsala, Sweden) and then centrifuged (1500 rpm, 30 min). The granulocyte fraction in the lowest layer was hemolyzed with cold purified water. Then, an eosinophil fraction was obtained by negative selection using anti-CD16 antibody-immobilized magnetic beads (MACS CD16 microbeads, Miltenyi Biotec, Bergisch Gladbach, Germany). The viability (trypan blue dye exclusion, Nakarai) and the purity (DIFF-QUICK staining, International Reagents Corporation) of eosinophils were both 99% or more.

Isolated human eosinophils were prepared at 2×10⁵ cells/tube and reacted with 6 mouse anti-human CD30 monoclonal antibodies or a control mouse IgG (10 µg/ml) as primary antibodies at 4°C for 30 min. After washing, the cells were stained with an FITC-labeled goat anti-mouse IgG F(ab')₂ as a secondary antibody (reacted at 4°C, 30 min) and fixed with 1% paraformaldehyde and then detected by a flow cytometer(FACScan Becton Dickinson). Eosinophils activated with IL-5 (1 ng/ml, R&D systems, Inc., Minneapolis, MN) (37°C, 30 min) were also tested in the same manner. The mouse anti-human CD30 monoclonal antibodies used were HRS-4 (Immunotech, Marseilles, France), Ber-H2 (DAKO, Glostrup, Denmark), Ber-H8 (PharMingen, San Diego, CA), AC-10 (Ancel, Bayport, MN), 1G12 (YLEM Avezzano, Roma, Italy) and Ki-1 (YLEM Avezzano, Roma, Italy), and the control mouse IgG used was MOPC-21 (Sigma Chemical Co., St. Louis, MO).

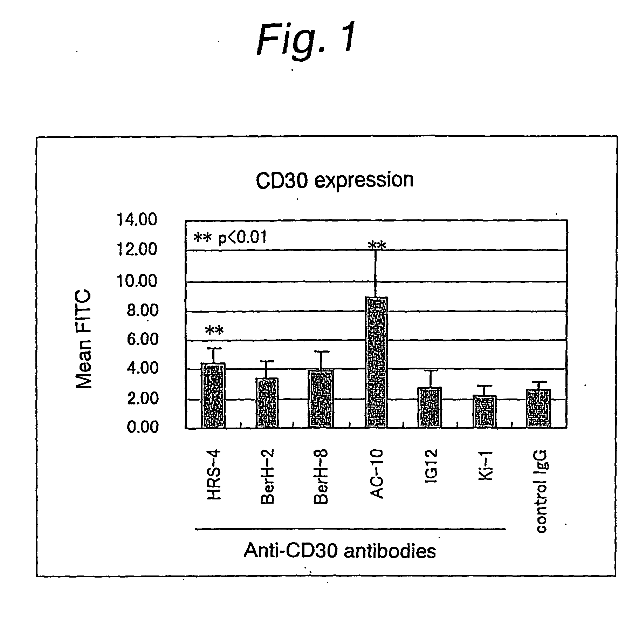

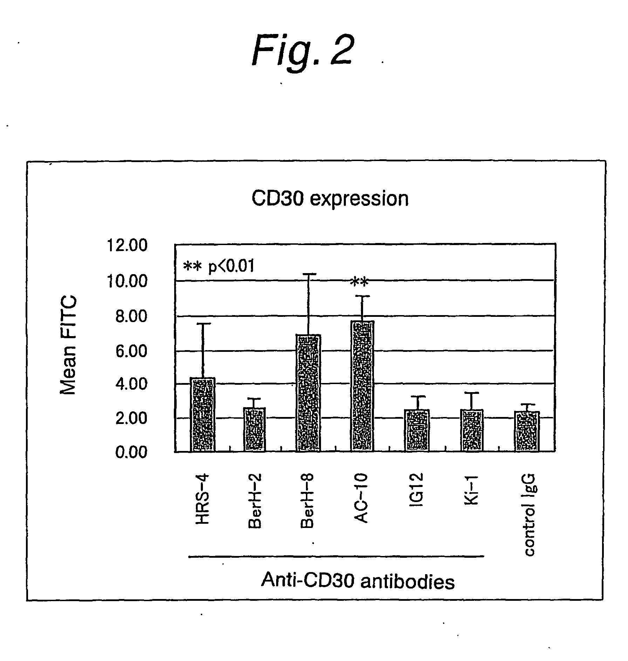

Flow cytometric analysis of eosinophils from 6 peripheral blood donors showed that human peripheral blood eosinophils had been significantly stained with HRS-4 and AC10, confirming CD30 expression (Fig. 1). The average fluorescence intensity by AC10 staining was 8.9 ± 3.2, showing that CD30 expression in eosinophils was comparable to the expression of IL-5 receptor α-chain. When the cells were activated with IL-5, AC10 showed a significant expression and HRS-4 or Ber-H8 showed a relatively increased expression (Fig. 2).

### Example 2: Analysis of CD30 mRNA expression in human peripheral blood eosinophils

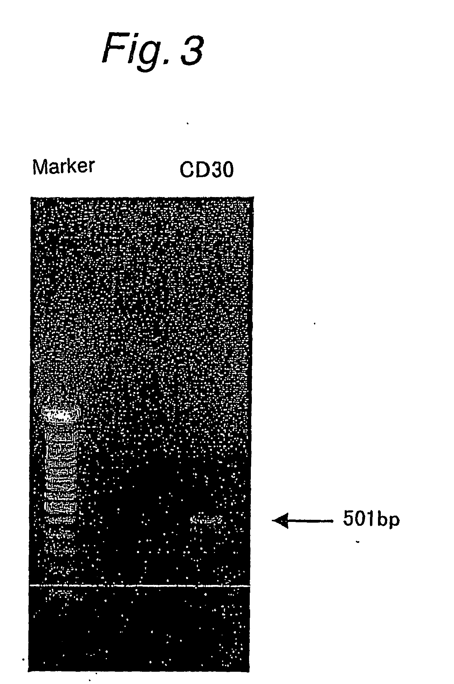

Human peripheral blood eosinophils were dissolved in Isogen (Nippon Gene, Osaka, Japan) to extract RNA, which was then reversely transcribed into cDNA using a kit from Invitrogen Corp (San Diego, CA). An upstream sense primer (5'-GCC CAG GAT CAA GTC ACT CAT-3') (SEQ ID NO: 3) and a downstream antisense primer (5'-TAC ACG TCT GAA GGC CCT AGG-3') (SEQ ID NO: 4) recognizing the 3'-untranslated region of the human CD30 gene were used to amplify a 501 bp fragment by PCR in a thermal cycler (Gene Amp PCR System 9700; PE Biosystem) (1 cycle of denaturation at 94°C for 1 min, 40 cycles at 94°C for 1 min/55°C for 1 min/72°C for 2 min, and finally 1 cycle of extension at 72°C for 10 min). After the completion of the reaction, the reaction product was electrophoresed on a 0.8% agarose gel (BRL Life Technologies Inc.) containing 0.05% ethidium bromide (Sigma) to show a target PCR product as a band of about 500 bp. Thus, it was shown that CD30 mRNA is expressed in human peripheral blood eosinophils (Fig. 3).

### Example 3: Evaluation of apoptosis induction in human peripheral blood eosinophils by anti-human CD30 monoclonal antibodies

Anti-human CD30 monoclonal antibodies were prepared at various concentrations in PBS (0.01, 0.1, 1 and 10 µg/ml) and 1 ml of each preparation was added to a 24-well microplate (Costar Corp., Cambridge, MA) and then left standing at 4°C for 12 hours for immobilization. After washing, the plate was blocked with 2 ml of 1% human serum albumin (Sigma Chemical Co., St. Louis, MO) at 37°C for 2 hours. After washing, isolated human peripheral blood eosinophils prepared at 1x10⁶ cells/ml in IMDM (Iscove's modified Dulbecco's medium, GIBCO) containing 10% FCS, 10⁻⁵ M 2-mercaptoethanol (GIBCO), Penicillin/Streptomycin (GIBCO) and 1 ng/ml human recombinant IL-5 (R&D systems, Inc., Minneapolis, MN) were added. After incubation at 37°C for 4 hours, cells were harvested and washed, and then apoptotic cells were detected using a MEBCYTO-Apoptosis Kit (Medical Biological Laboratories, Nagoya, Japan). Apoptotic cells to which FITC-labeled Annexin V had been bound were measured by a flow cytometer (FACScan Becton Dickinson).

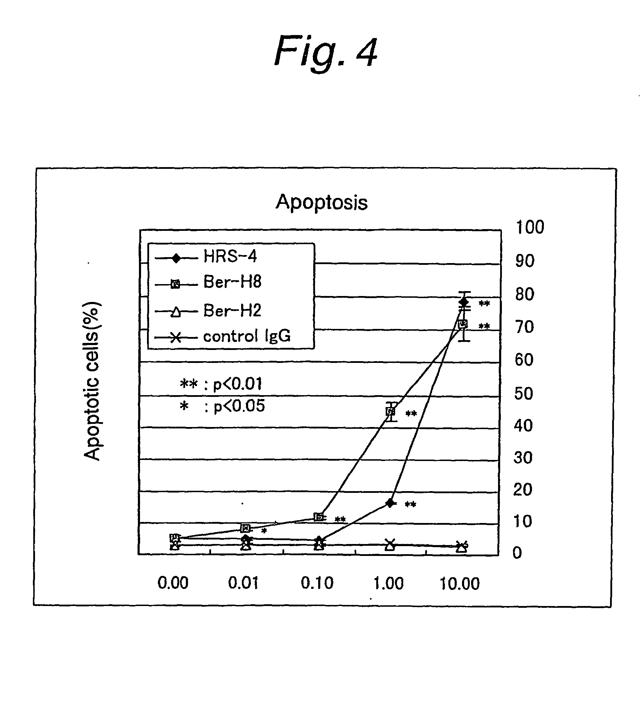

The results showed that anti-human CD30 monoclonal antibodies HRS-4 and Ber-H8 concentration-dependently induced apoptosis in human peripheral blood eosinophils. Apoptosis was induced in 16.3% and 78.8% of eosinophils by HRS-4 immobilized at concentrations of 1 and 10 µg/ml, respectively; and 7.8%, 11.4%, 44.8% and 71.9% of eosinophils by Ber-H8 immobilized at concentrations of 0.01, 0.1, 1 and 10 µg/ml, respectively (Fig. 4). However, anti-human CD30 monoclonal antibodies Ber-H2, AC10, 1G12 and Ki1 and the control mouse IgG induced no apoptosis in human peripheral blood eosinophils.

### Example 4: Evaluation of individual variations among eosinophil donors in the apoptosis induction in human peripheral blood eosinophils by anti-human CD30 monoclonal antibodies

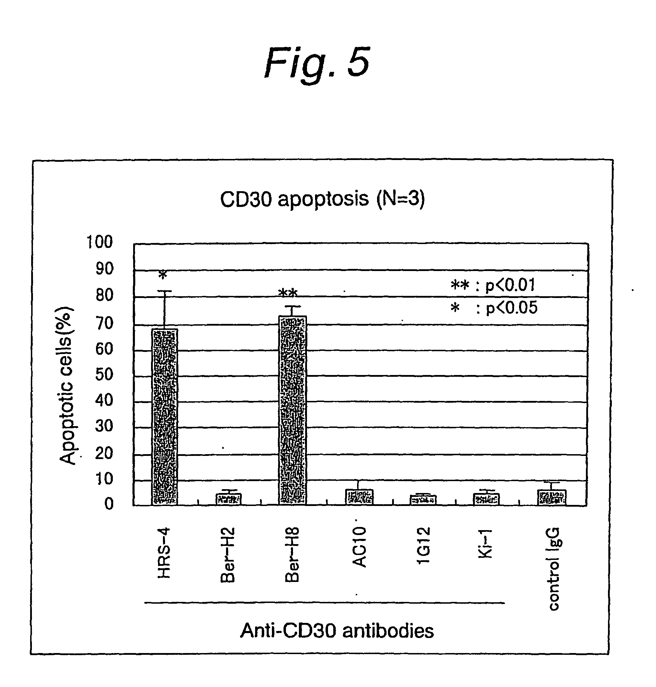

The ability of anti-human CD30 monoclonal antibodies to induce apoptosis in eosinophils isolated from peripheral blood of 3 donors was evaluated. Anti-human CD30 monoclonal antibodies were immobilized at a concentration of 10 µg/ml. Anti-human CD30 monoclonal antibodies HRS-4 and Ber-H8 strongly induced apoptosis in peripheral blood eosinophils from all the donors (Fig. 5 shows the averages of the 3 donors). Thus, it was shown that no individual variation occurs among eosinophil donors in the ability of HRS-4 and Ber-H8 to induce apoptosis in peripheral blood eosinophils. However, anti-human CD30 monoclonal antibodies Ber-H2, AC10, 1G12 and Ki1 and the control mouse IgG induced no apoptosis in eosinophils from any donors.

### Example 5: Time kinetics in the apoptosis induction in human peripheral blood eosinophils by anti-human CD30 monoclonal antibodies HRS-4 and Ber-H8

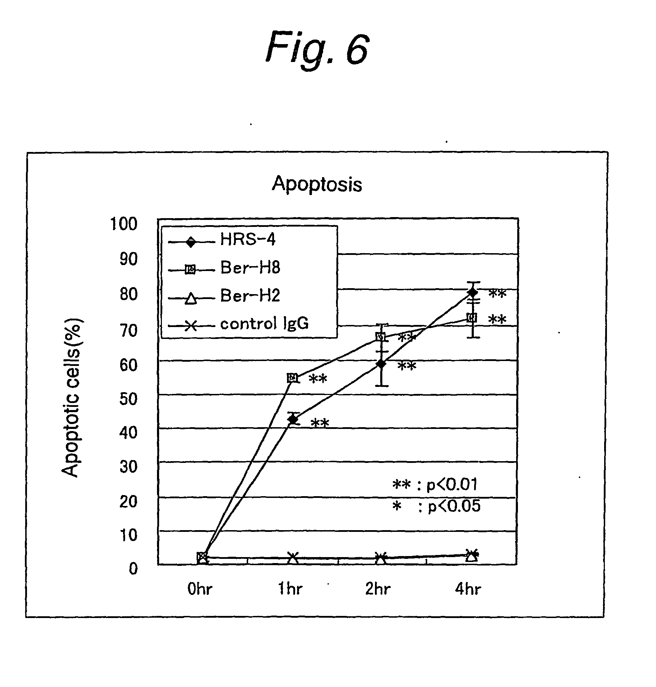

Time kinetics in the apoptosis induction in isolated human peripheral blood eosinophils by anti-human CD30 monoclonal antibodies HRS-4 and Ber-H8 were evaluated. When eosinophils were cultured on plates to which HRS-4 and Ber-H8 had been immobilized at a concentration of 10 µg/ml, both antibodies induced apoptosis in approximately 50% of eosinophils after 1 hour and apoptotic eosinophils increased to 70% after 4 hours (Fig. 6). Thus, HRS-4 and Ber-H8 were found to induce apoptosis very rapidly in peripheral blood eosinophils.

### Example 6: Apoptosis induction in human peripheral blood eosinophils by anti-human CD30 monoclonal antibodies HRS-4 and Ber-H8 (based on DNA fragmentation)

Apoptosis induction in isolated human peripheral blood eosinophils by anti-human CD30 monoclonal antibodies HRS-4 and Ber-H8 was evaluated on the basis of DNA fragmentation. After eosinophils were cultured for 24 hours on plates to which HRS-4 and Ber-H8 had been immobilized at a concentration of 10 µg/ml, cells were harvested and DNA was extracted using an apoptosis ladder detection kit (Wako Pure Chemical Industries, Ltd., Osaka, Japan) to evaluate DNA fragmentation by agarose electrophoresis.

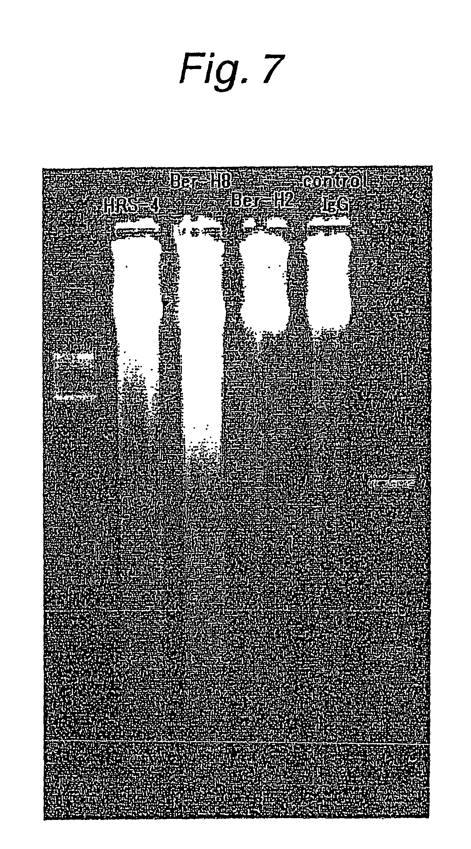

Both HRS-4 and Ber-H8 induced fragmentation of eosinophilic DNA (Fig. 7). However, neither anti-human CD30 monoclonal antibody Ber-H2 nor control mouse IgG induced fragmentation of eosinophilic DNA. Thus, it could be confirmed by DNA fragmentation that HRS-4 and Ber-H8 induce apoptosis in human peripheral blood eosinophils.

### Example 7: Evaluation of the ability of anti-human CD30 monoclonal antibodies HRS-4 and Ber-H8 to induce apoptosis in human peripheral blood eosinophils in comparison with an anti-human Fas monoclonal antibody

The ability of anti-human CD30 monoclonal antibodies HRS-4 and Ber-H8 to induce apoptosis in isolated human peripheral blood eosinophils was evaluated in comparison with an anti-human Fas monoclonal antibody (CH-11, Medical Biological Laboratories, Nagoya, Japan). Eosinophils were cultured on plates to which the monoclonal antibodies had been immobilized at a concentration of 10 µg/ml.

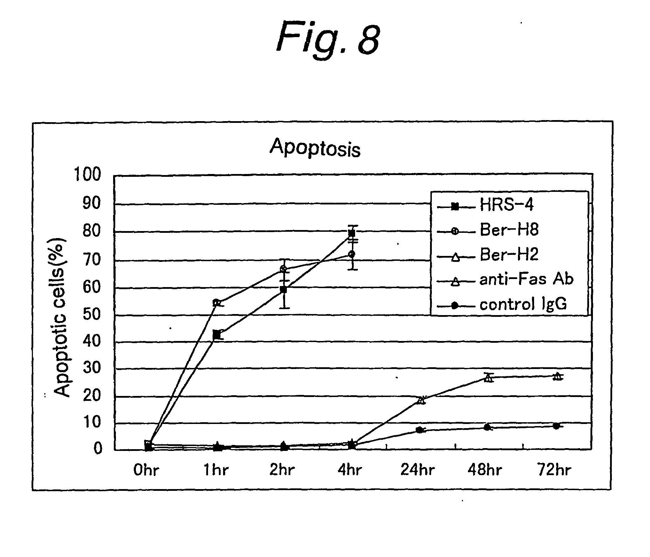

HRS-4 and Ber-H8 induced apoptosis in approximately 50% of eosinophils after incubation for 1 hour and apoptotic eosinophils increased to 70% after 4 hours. When the anti-human Fas monoclonal antibody was used, apoptotic eosinophils appeared on and after 24 hours of incubation but increased slightly even after 72 hours (Fig. 8). Thus, anti-human CD30 monoclonal antibodies HRS-4 and Ber-H8 were found to induce apoptosis very rapidly and strongly in human peripheral blood eosinophils as compared with the anti-human Fas monoclonal antibody.

### Example 8: Evaluation of cell selectivity of the apoptosis-inducing ability of anti-human CD30 monoclonal antibodies HRS-4 and Ber-H8

Heparinized human peripheral blood was diluted with PBS and layered over Percoll (density 1.090 g/ml, Pharmacia, Uppsala, Sweden) and then centrifuged (1500 rpm, 15 min). The granulocyte fraction in the lowest layer was hemolyzed with cold purified water. Then, a neutrophil fraction was obtained by positive selection using anti-CD16 antibody-immobilized magnetic beads (MACS CD16 microbeads, Miltenyi Biotec, Bergisch Gladbach, Germany). The viability of neutrophils was 99%. The ability of the immobilized anti-human CD30 monoclonal antibodies to induce apoptosis in isolated human peripheral blood neutrophils was assessed by a flow cytometer.

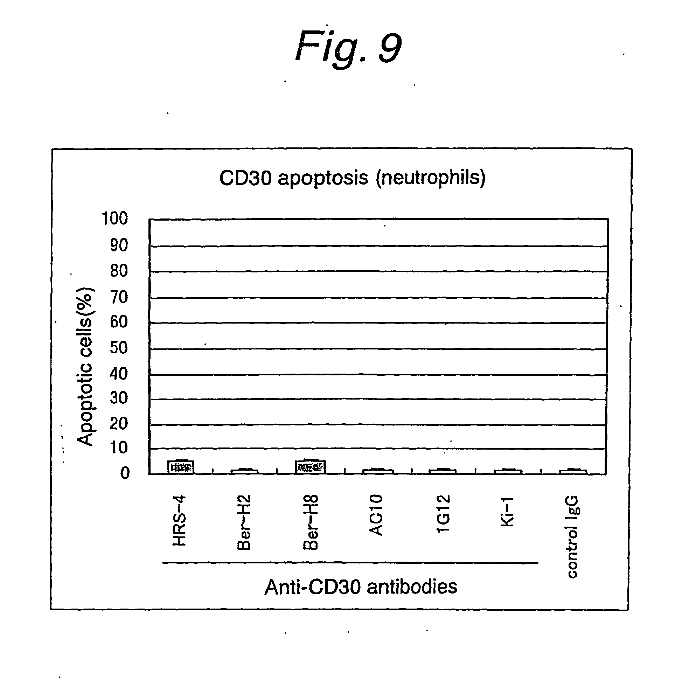

None of the above 6 anti-human CD30 monoclonal antibodies induced apoptosis in neutrophils (Fig. 9). Further evaluation was made using peripheral blood neutrophils from 6 donors, but none of the 6 anti-human CD30 monoclonal antibodies induced apoptosis.

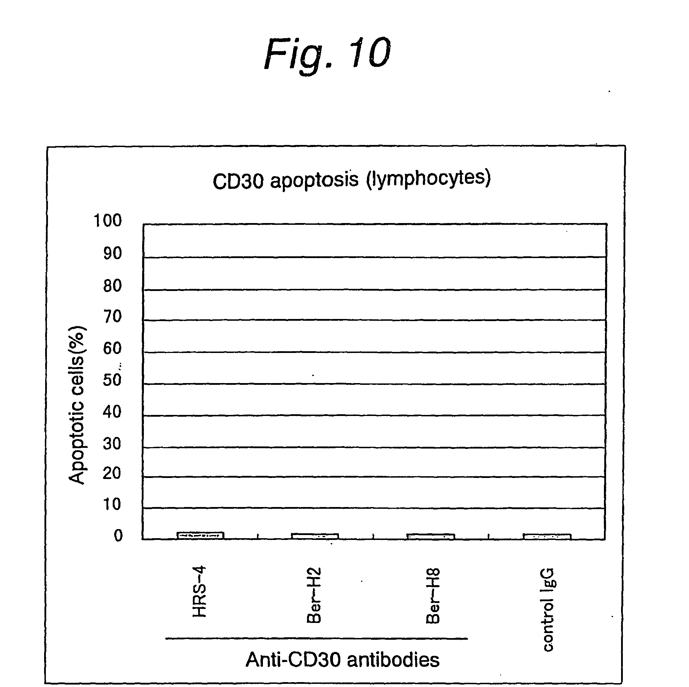

Then, heparinized human peripheral blood was depleted of macrophages by the action of silica gel (IBL Co., Ltd.) at 37°C for 1 hour, and diluted with PBS and then layered over LSM (density 1.077 g/ml, ICN), and then centrifuged (1500 rpm, 15 min) to give a lymphocyte fraction. The viability of lymphocytes was 99%. The ability of the immobilized anti-human CD30 monoclonal antibodies to induce apoptosis in isolated human peripheral blood lymphocytes was measured by a flow cytometer in the same manner. Neither HRS-4 nor Ber-H8 induced apoptosis in lymphocytes (Fig. 10) though both induced apoptosis in eosinophils.

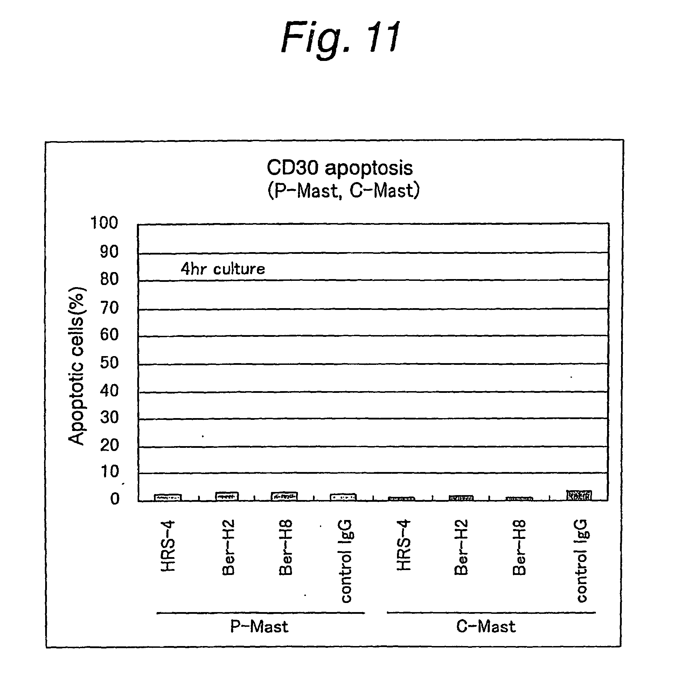

The ability of the immobilized anti-human CD30 monoclonal antibodies to induce apoptosis in cultured human mast cells was also measured by a flow cytometer. Neither HRS-4 nor Ber-H8 induced apoptosis in cultured human mast cells (Fig. 11) though both induced apoptosis in eosinophils. The cultured human mast cells used were peripheral blood-derived mature mast cells (P-Mast) and cord blood-derived mature mast cells (C-Mast) obtained according to the method of Saito et al. (J. Immunol. 157, 343-350, 1996 and J. Allergy Clin. Immunol. 106, 321-328, 2000).

Moreover, neither HRS-4 nor Ber-H8 induced apoptosis in CD30-positive human cell lines Jurkat (T cell lymphoma), K562 (erythroleukemia) and RD (rhabdomyosarcoma).

Thus, anti-human CD30 monoclonal antibodies HRS-4 and Ber-H8 were found to induce apoptosis selectively in human peripheral blood eosinophils.

### Example 9: Influence of high-concentration IL-5 on the apoptosis induction in human peripheral blood eosinophils by anti-human CD30 monoclonal antibodies HRS-4 and Ber-H8

Previous evidence showed that anti-human CD30 monoclonal antibodies HRS-4 and Ber-H8 induced apoptosis in human peripheral blood eosinophils in the presence of 10 ng/ml of human recombinant IL-5. However, the viability of eosinophils themselves may increase to diminish the apoptosis-inducing ability of HRS-4 and Ber-H8 if IL-5 is present at high concentrations. Thus, the ability of HRS-4 and Ber-H8 to induce apoptosis in human peripheral blood eosinophils was evaluated in the presence of 0, 1, 10 and 100 ng/ml of IL-5. Taking into account individual variations among eosinophil donors, peripheral blood eosinophils from 4 donors were used.

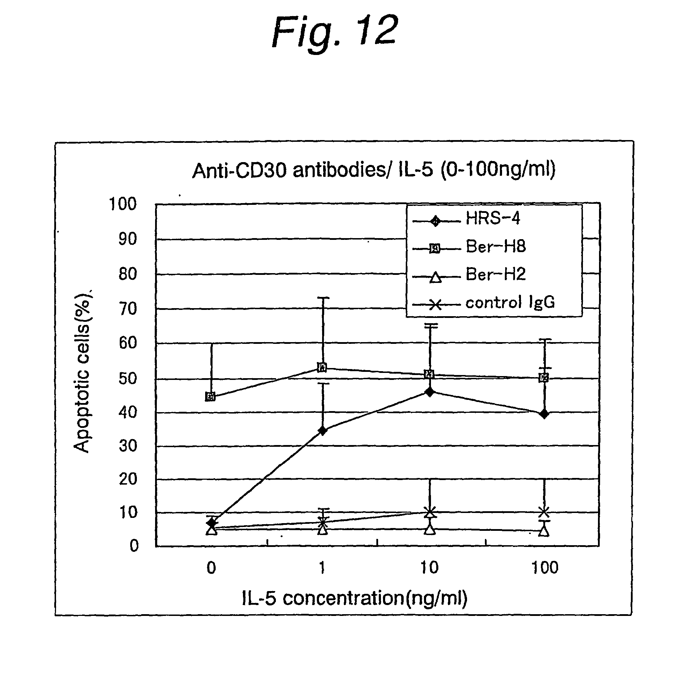

HRS-4 induced apoptosis IL-5 concentration-dependently and no apoptosis in the absence of IL-5, but it induced apoptosis even at 100 ng/ml. However, Ber-H8 induced apoptosis whether IL-5 was present or not (Fig. 12).

This suggested that CD30-mediated apoptosis in human peripheral blood eosinophils occurs via signaling pathways in which IL-5 is involved and not. However, it was shown that apoptosis induction by HRS-4 and Ber-H8 was not diminished even in the presence of at least high concentrations of IL-5.

### Example 10: Evaluation of apoptosis induction in human peripheral blood eosinophils by anti-mouse CD30 monoclonal antibodies

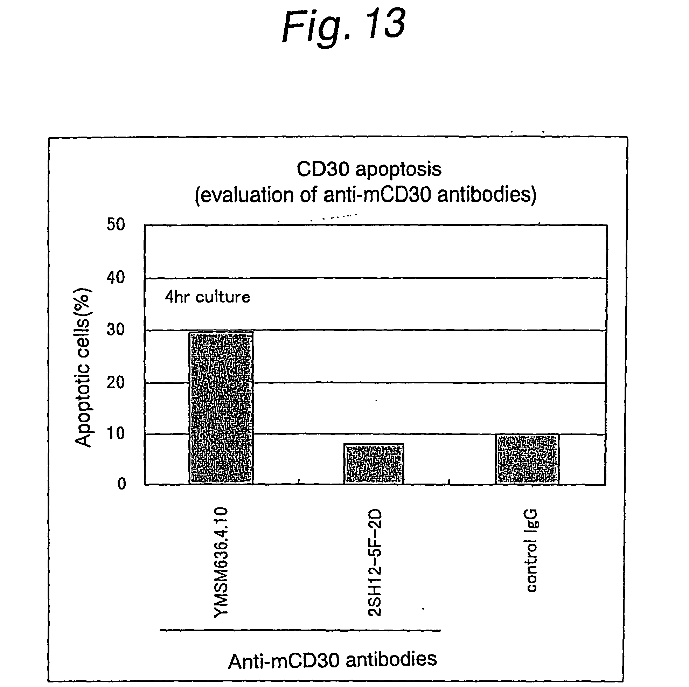

Apoptosis induction in isolated human peripheral blood eosinophils by anti-mouse CD30 monoclonal antibodies YMSM636.4.10 (Serotec Ltd, Oxford UK) and 2SH12-5F-2D (BD PharMingen, San Diego, USA) was evaluated. When human eosinophils were cultured on a plate to which YMSM636.4.10 had been immobilized at a concentration of 10 µg/ml, apoptosis was induced in approximately 30% of eosinophils after 4 hours (Fig. 13). However, 2SH12-5F-2D and a rat control IgG induced no apoptosis.

### Example 11: Evaluation of apoptosis induction in human peripheral blood eosinophils by a mouse CD30 ligand

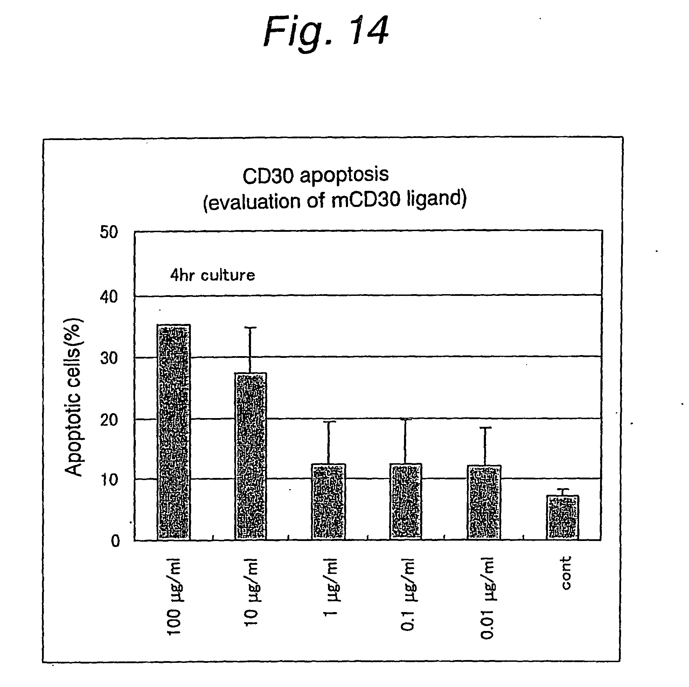

Apoptosis induction in isolated human peripheral blood eosinophils by a recombinant mouse CD30 ligand (R&D Systems Inc. Minneapolis, USA) was evaluated. When human eosinophils were cultured on plates to which the recombinant mouse CD30 ligand had been immobilized at concentrations of 10 and 100 µg/ml, apoptosis was induced in 28% and 35% of eosinophils after 4 hours (Fig. 14).

### Example 12: Evaluation of apoptosis induction in human peripheral blood eosinophils by anti-human CD30 monoclonal antibodies HRS-4 and Ber-H8 immobilized on polystyrene microbeads

Apoptosis induction in isolated human peripheral blood eosinophils by anti-human CD30 monoclonal antibodies HRS-4 and Ber-H8 immobilized on polystyrene microbeads was evaluated. According to the method of Kita et al. (Am. J. Respir. Cell. Mol. Biol., 18: 675-686, 1998), 200 µg of the anti-human CD30 monoclonal antibodies were added to 0.5 ml of polystyrene microbeads having a diameter of 1.444 µM (2.56% solid Latex, Sigma) washed with 0.1 M borate buffer and left standing at 4°C for 12 hours for immobilization. After washing, the beads were blocked with 0.5 ml of 2.5% human serum albumin at 4°C for 2 hours. After washing, the beads were suspended in 0.5 ml of PBS to prepare anti-human CD30 monoclonal antibodies immobilized on polystyrene microbeads. To 96-well U-bottomed microplates (NUNC) were added 2x10⁵ cells of isolated human peripheral blood eosinophils and HRS-4 and Ber-H8 immobilized on polystyrene microbeads (10 µl). After incubation at 37°C for 4 hours, apoptotic cells were measured by a flow cytometer. Signals from polystyrene microbeads were excluded by gating on the forward versus side scatter dot plot.

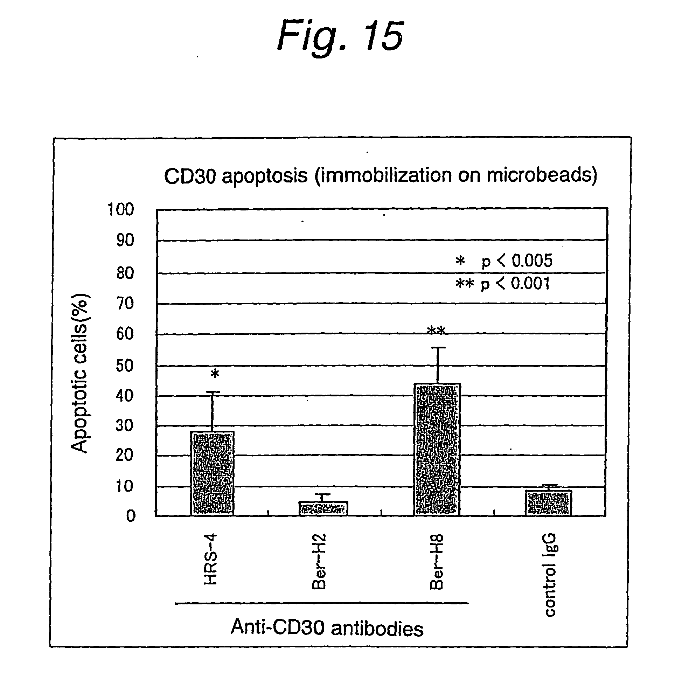

HRS-4 and Ber-H8 immobilized on polystyrene microbeads induced apoptosis in 27.9% and 44.0% of eosinophils, respectively (Fig. 15). However, anti-human CD30 monoclonal antibody Ber-H2 and the control mouse IgG immobilized on polystyrene microbeads did not induce apoptosis in human peripheral blood eosinophils. Thus, anti-human CD30 monoclonal antibodies HRS-4 and Ber-H8 were found to also induce apoptosis in human eosinophils when they were immobilized on polystyrene microbeads in the same manner as they were immobilized on microplates.

### Example 13: Phagocytosis assay by phagocytes on human peripheral blood eosinophils in which apoptosis was induced by anti-human CD30 monoclonal antibodies HRS-4 and Ber-H8 immobilized on polystyrene microbeads

A phagocytosis assay by phagocytes was performed on isolated eosinophils in which apoptosis was induced by HRS-4 and Ber-H8 immobilized on polystyrene microbeads. According to the method of Brown et al. (J. Biol. Chem., 275: 5987-5996, 2000), CD34-negative cells were first fractionated from a human cord blood mononuclear fraction and cultured in the presence of 10 ng/ml of human recombinant M-CSF (Macrophage-colony stimulating factor, R&D) for 7 days to prepare mature macrophages. Then, eosinophils were reacted with HRS-4 and Ber-H8 immobilized on polystyrene microbeads for 30 minutes and, during apoptosis induction, they were assayed for phagocytosis by cord blood-derived macrophages precultured on chamber slides (NUNC) for 24 hours according to the method of Fadok et al. (J. Biol. Chem., 276: 1071-1077, 2001). Phagocytic effect was evaluated on the basis of the difference of the concentrations of an eosinophil granule protein EDN (Eosinophil-derived neurotoxin) secreted in the culture supernatants of eosinophil monocultures and eosinophil /macrophage cocultures after 4 hours. The EDN concentrations were measured using an ELISA kit (MBL).

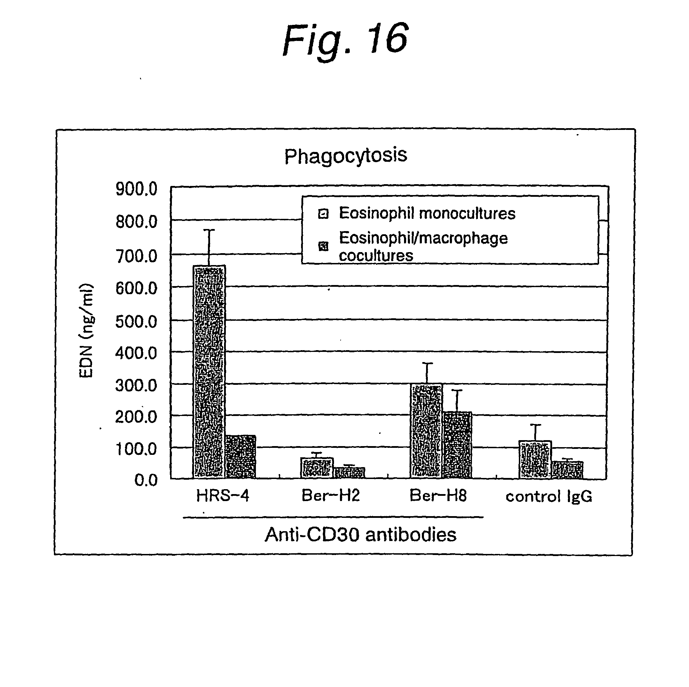

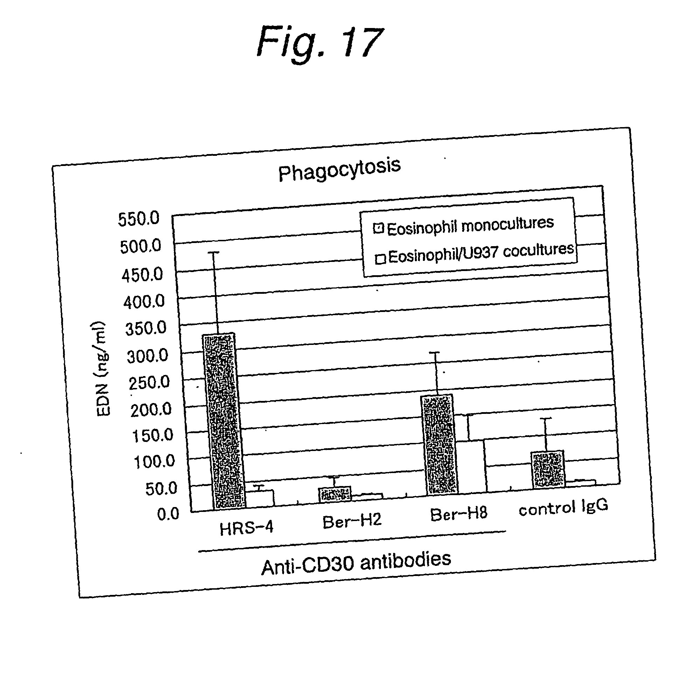

Eosinophils in which apoptosis was induced by HRS-4 and Ber-H8 immobilized on polystyrene microbeads showed a decrease in EDN secreted in the culture supernatants as a result of phagocytosis by cocultures with cord blood-derived macrophages. Especially, cord blood-derived macrophages showed a remarkable phagocytic effect on eosinophils in which apoptosis was induced by HRS-4 (Fig. 16). However, both monocultures and cocultures of eosinophils reacted with anti-human CD30 monoclonal antibody Ber-H2 and the control mouse IgG immobilized on polystyrene microbeads showed very low EDN secretion. Similar results were obtained in a phagocytosis assay using a human monocytic cell line U937 (e.g. available from ATCC under ATCC No. CRL-1593.2) activated by stimulation with 10 ng/ml PMA (Phorbol 12-Myristate 13-Acetate, Sigma) for 1 hour for the purpose of eliciting the function as macrophages to trigger their phagocytic ability (Fig. 17).

This suggested that human peripheral blood eosinophils in which apoptosis was induced by HRS-4 and Ber-H8 are phagocytized by phagocytes such as macrophages before granule proteins are secreted by the destruction of cell membranes.

## Claims

1. A therapeutic agent for a hypereosinophilic disease comprising a substance inducing eosinophil apoptosis via CD30 and/or a molecule involved in CD30 signal transduction as an active ingredient.

2. The therapeutic agent for a hypereosinophilic disease as defined in claim 1 wherein the substance inducing eosinophil apoptosis via CD30 and/or a molecule involved in CD30 signal transduction is an immunoglobulin recognizing site recognized by Ber-H8 or HRS-4 or an active fragment, peptide or low-molecular weight compound thereof.

3. The therapeutic agent for a hypereosinophilic disease as defined in claim 1 wherein the substance inducing eosinophil apoptosis via CD30 and/or a molecule involved in CD30 signal transduction is an immunoglobulin or an active fragment thereof.

4. The therapeutic agent for a hypereosinophilic disease as defined in claim 2 wherein the substance inducing eosinophil apoptosis via CD30 and/or a molecule involved in CD30 signal transduction is an immunoglobulin recognizing at least a part or the whole of the amino acid sequence of SEQ ID NO: 1 (the amino acid sequence between positions 112 and 412 of the amino acid sequence of CD30) or an active fragment thereof.

5. The therapeutic agent for a hypereosinophilic disease as defined in claim 4 wherein the immunoglobulin is Ber-H8 or HRS-4.

6. The therapeutic agent for a hypereosinophilic disease as defined in claim 1 wherein the substance inducing eosinophil apoptosis via CD30 and/or a molecule involved in CD30 signal transduction is a CD30 ligand or an active fragment thereof.

7. The therapeutic agent for a hypereosinophilic disease as defined in claim 1 wherein the substance inducing eosinophil apoptosis via CD30 and/or a molecule involved in CD30 signal transduction is a nucleic acid encoding a CD30 ligand or an active fragment thereof.

8. The therapeutic agent for a hypereosinophilic disease as defined in claim 1 wherein the hypereosinophilic disease is a disease selected from the group consisting of allergic disease, respiratory disease, skin disease, autoimmune disease, immunodeficiency disease, digestive disease, tumorous disease or parasitic infection.

9. The therapeutic agent for a hypereosinophilic disease as defined in claim 8 wherein the allergic disease is a disease selected from the group consisting of drug allergy, bronchial asthma and allergic rhinitis.


[SEQUENCE LISTING]
<110> SUNTORY LIMITED
SUNTORY BIOMEDICAL RESEARCH LIMITED
<120> REMEDIES FOR EOSINOPHILIC DISEASES
<130> 011472
<160> 5

<210> 1
<211> 595
<212> PRT
<213> Homo sapiens
<400> 1
Met Arg Val Leu Leu Ala Ala Leu Gly Leu Leu Phe Leu Gly Ala Leu
                5                   10                  15
Arg Ala Phe Pro Gln Asp Arg Pro Phe Glu Asp Thr Cys His Gly Asn
            20                  25                  30
Pro Ser His Tyr Tyr Asp Lys Ala Val Arg Arg Cys Cys Tyr Arg Cys
        35                  40                  45
Pro Met Gly Leu Phe Pro Thr Gln Gln Cys Pro Gln Arg Pro Thr Asp
    50                  55                  60
Cys Arg Lys Gln Cys Glu Pro Asp Tyr Tyr Leu Asp Glu Ala Asp Arg
65                  70                  75                  80
Cys Thr Ala Cys Val Thr Cys Ser Arg Asp Asp Leu Val Glu Lys Thr
                85                  90                  95
Pro Cys Ala Trp Asn Ser Ser Arg Val Cys Glu Cys Arg Pro Gly Met
            100                 105                 110
Phe Cys Ser Thr Ser Ala Val Asn Ser Cys Ala Arg Cys Phe Phe His
        115                 120                 125
Ser Val Cys Pro Ala Gly Met Ile Val Lys Phe Pro Gly Thr Ala Gln

130                 135                 140

Lys Asn Thr Val Cys Glu Pro Ala Ser Pro Gly Val Ser Pro Ala Cys

145                 150                 155                 160

Ala Ser Pro Glu Asn Cys Lys Glu Pro Ser Ser Gly Thr Ile Pro Gln

                165                 170                 175

Ala Lys Pro Thr Pro Val Ser Pro Ala Thr Ser Ser Ala Ser Thr Met

            180                 185                 190

Pro Val Arg Gly Gly Thr Arg Leu Ala Gln Glu Ala Ala Ser Lys Leu

        195                 200                 205

Thr Arg Ala Pro Asp Ser Pro Ser Ser Val Gly Arg Pro Ser Ser Asp

    210                 215                 220

Pro Gly Leu Ser Pro Thr Gln Pro Cys Pro Glu Gly Ser Gly Asp Cys

225                 230                 235                 240

Arg Lys Gln Cys Glu Pro Asp Tyr Tyr Leu Asp Glu Ala Gly Arg Cys

                245                 250                 255

Thr Ala Cys Val Ser Cys Ser Arg Asp Asp Leu Val Glu Lys Thr Pro

            260                 265                 270

Cys Ala Trp Asn Ser Ser Arg Thr Cys Glu Cys Arg Pro Gly Met Ile

        275                 280                 285

Cys Ala Thr Ser Ala Thr Asn Ser Cys Ala Arg Cys Val Pro Tyr Pro

    290                 295                 300

Ile Cys Ala Ala Glu Thr Val Thr Lys Pro Gln Asp Met Ala Glu Lys

305                 310                 315                 320

Asp Thr Thr Phe Glu Ala Pro Pro Leu Gly Thr Gln Pro Asp Cys Asn

                325                 330                 335

Pro Thr Pro Glu Asn Gly Glu Ala Pro Ala Ser Thr Ser Pro Thr Gln

            340                 345                 350

Ser Leu Leu Val Asp Ser Gln Ala Ser Lys Thr Leu Pro Ile Pro Thr

        355                 360                 365

Ser Ala Pro Val Ala Leu Ser Ser Thr Gly Lys Pro Val Leu Asp Ala
    370                 375                 380
Gly Pro Val Leu Phe Trp Val Ile Leu Val Leu Val Val Val Val Gly
385                 390                 395                 400
Ser Ser Ala Phe Leu Leu Cys His Arg Arg Ala Cys Arg Lys Arg Ile
                405                 410                 415
Arg Gln Lys Leu His Leu Cys Tyr Pro Val Gln Thr Ser Gln Pro Lys
            420                 425                 430
Leu Glu Leu Val Asp Ser Arg Pro Arg Arg Ser Ser Thr Gln Leu Arg
        435                 440                 445
Ser Gly Ala Ser Val Thr Glu Pro Val Ala Glu Glu Arg Gly Leu Met
    450                 455                 460
Ser Gln Pro Leu Met Glu Thr Cys His Ser Val Gly Ala Ala Tyr Leu
465                 470                 475                 480
Glu Ser Leu Pro Leu Gln Asp Ala Ser Pro Ala Gly Gly Pro Ser Ser
                485                 490                 495
Pro Arg Asp Leu Pro Glu Pro Arg Val Ser Thr Glu His Thr Asn Asn
            500                 505                 510
Lys Ile Glu Lys Ile Tyr Ile Met Lys Ala Asp Thr Val Ile Val Gly
        515                 520                 525
Thr Val Lys Ala Glu Leu Pro Glu Gly Arg Gly Leu Ala Gly Pro Ala
    530                 535                 540
Glu Pro Glu Leu Glu Glu Glu Leu Glu Ala Asp His Thr Pro His Tyr
545                 550                 555                 560
Pro Glu Gln Glu Thr Glu Pro Pro Leu Gly Ser Cys Ser Asp Val Met
                565                 570                 575
Leu Ser Val Glu Glu Glu Gly Lys Glu Asp Pro Leu Pro Thr Ala Ala
            580                 585                 590
Ser Gly Lys

        595

<210> 2
<211> 234
<212> PRT
<213> Homo sapiens
<400> 2
Met Asp Pro Gly Leu Gln Gln Ala Leu Asn Gly Met Ala Pro Pro Gly
                5                   10                  15
Asp Thr Ala Met His Val Pro Ala Gly Ser Val Ala Ser His Leu Gly
            20                  25                  30
Thr Thr Ser Arg Ser Tyr Phe Tyr Leu Thr Thr Ala Thr Leu Ala Leu
        35                  40                  45
Cys Leu Val Phe Thr Val Ala Thr Ile Met Val Leu Val Val Gln Arg
    50                  55                  60
Thr Asp Ser Ile Pro Asn Ser Pro Asp Asn Val Pro Leu Lys Gly Gly
65                  70                  75                  80
Asn Cys Ser Glu Asp Leu Leu Cys Ile Leu Lys Arg Ala Pro Phe Lys
                85                  90                  95
Lys Ser Trp Ala Tyr Leu Gln Val Ala Lys His Leu Asn Lys Thr Lys
            100                 105                 110
Leu Ser Trp Asn Lys Asp Gly Ile Leu His Gly Val Arg Tyr Gln Asp
        115                 120                 125
Gly Asn Leu Val Ile Gln Phe Pro Gly Leu Tyr Phe Ile Ile Cys Gln
    130                 135                 140
Leu Gln Phe Leu Val Gln Cys Pro Asn Asn Ser Val Asp Leu Lys Leu
145                 150                 155                 160
Glu Leu Leu Ile Asn Lys His Ile Lys Lys Gln Ala Leu Val Thr Val
                165                 170                 175

Cys Glu Ser Gly Met Gln Thr Lys His Val Tyr Gln Asn Leu Ser Gln
            180                 185                 190
Phe Leu Leu Asp Tyr Leu Gln Val Asn Thr Thr Ile Ser Val Asn Val
        195                 200                 205
Asp Thr Phe Gln Tyr Ile Asp Thr Ser Thr Phe Pro Leu Glu Asn Val
    210                 215                 220
Leu Ser Ile Phe Leu Tyr Ser Asn Ser Asp
225                 230

<210> 3
<211> 21
<212> DNA
<213> Artificial Sequence
<400> 3
gcccaggatc aagtcactca t

<210> 4
<211> 21
<212> DNA
<213> Artificial Sequence
<400> 4
tacacgtctg aaggccctag g

<210> 5
<211> 239
<212> PRT
<213> Mouse
<400> 5
Met Glu Pro Gly Leu Gln Gln Ala Gly Ser Cys Gly Ala Pro Ser Pro

                5                   10                  15
Asp Pro Ala Met Gln Val Gln Pro Gly Ser Val Ala Ser Pro Trp Arg
            20                  25                  30
Ser Thr Arg Pro Trp Arg Ser Thr Ser Arg Ser Tyr Phe Tyr Leu Ser
        35                  40                  45
Thr Thr Ala Leu Val Cys Leu Val Val Ala Val Ala Ile Ile Leu Val
    50                  55                  60
Leu Val Val Gln Lys Lys Asp Ser Thr Pro Asn Thr Thr Glu Lys Ala
65                  70                  75                  80
Pro Leu Lys Gly Gly Asn Cys Ser Glu Asp Leu Phe Cys Thr Leu Lys
                85                  90                  95
Ser Thr Pro Ser Lys Lys Ser Trp Ala Tyr Leu Gln Val Ser Lys His
            100                 105                 110
Leu Asn Asn Thr Lys Leu Ser Trp Asn Glu Asp Gly Thr Ile His Gly
        115                 120                 125
Leu Ile Tyr Gln Asp Gly Asn Leu Ile Val Gln Phe Pro Gly Leu Tyr
    130                 135                 140
Phe Ile Val Cys Gln Leu Gln Phe Leu Val Gln Cys Ser Asn His Ser
145                 150                 155                 160
Val Asp Leu Thr Leu Gln Leu Leu Ile Asn Ser Lys Ile Lys Lys Gln
                165                 170                 175
Thr Leu Val Thr Val Cys Glu Ser Gly Val Gln Ser Lys Asn Ile Tyr
            180                 185                 190
Gln Asn Leu Ser Gln Phe Leu Leu His Tyr Leu Gln Val Asn Ser Thr
        195                 200                 205
Ile Ser Val Arg Val Asp Asn Phe Gln Tyr Val Asp Thr Asn Thr Phe
    210                 215                 220
Pro Leu Asp Asn Val Leu Ser Val Phe Leu Tyr Ser Ser Ser Asp
225                 230                 235